# DEMANDE DE BREVET EUROPEEN

(11) **EP 4 796 049 A1**
(43) Date de publication de la demande: **26.08.2026**
(21) Numéro de dépôt: 26159159.8
(22) Date de dépôt: 17.02.2026
(51) Int. Cl.: A61B 10/00, G01N 31/22, G01N 33/493, G01N 35/00, G01N 35/10

(54) **CONTRÔLE DE L`INJECTION D`UN LIQUIDE SUR UN SUPPORT DE TEST**

(30) Priorité: 20.02.2025 FR 2501746
(71) Demandeur: Withings, 92130 Issy-Les-Moulineaux (FR)
(72) Inventeur: Merlot, Antoine, 92130 Issy-les-Moulineaux (FR); Brac de la Perrière, Brice, 92130 Issy-les-Moulineaux (FR)
(74) Mandataire: Withings IP

(57) **Abrégé**

La présente description concerne un procédé de contrôle d'injection d'un liquide sur un support test (312), le support de test comprenant un matériau absorbant apte à absorber le liquide et un réactif apte à réagir avec au moins un composant présent dans le liquide, le procédé étant mis en œuvre à l'aide d'un injecteur et d'un capteur optique.

## Description

La présente invention concerne l'analyse automatique de supports de test, notamment pour des tests de liquides corporelles (par exemple de l'urine). L'analyse automatique peut être mise en œuvre dans un dispositif d'analyse d'urine « à la maison », appelé aussi « *point* of *care* » en anglais, et non pas sur du matériel de laboratoire ou d'hôpital. Les supports de test prennent généralement la forme de bandelettes.

### Etat de la technique

Lors de l'analyse d'un support de test, il est nécessaire de savoir si la bandelette a été correctement imbibée par le fluide concerné (urine, sang, salive, etc.). Pour des bandelettes dites « *lateral flow* » ( « flux latéral » en français mais connues sous le nom d'immunochromatographique), des lignes de contrôle sont généralement prévues. Le document EP2385369 utilise un détecteur optique pour repérer la ligne de contrôle qui peut apparaitre. Toutefois, pour des bandelettes colorimétriques qui intègrent des pads (ou tampons) colorimétriques, des lignes de contrôle ne sont pas nécessaires, notamment parce que le pad colorimétrique est souvent directement imbibé par dépôt de liquide sur ce dernier. Dans une bandelette de type *lateral flow*, un déplacement d'un front de liquide entraine une réaction dans une zone de test et dans une zone de contrôle, au-delà de la zone de test, où la ligne de contrôle se situe. Sur un support de test colorimétrique traditionnel, il n'y a pas de déplacement d'urine.

Toutefois, dans un objectif d'automatisation, il est préférable d'avoir un dispositif fonctionnant aussi bien sur des supports de test colorimétriques que des supports de test de type *lateral flow.* De la même façon, dans un optique d'une dispositif personnel, utilisable à la maison, il est préférable d'avoir un dispositif compact et robuste. Or, ces dispositifs peuvent présenter des difficultés liées au débit d'un injecteur, qui peut être variable, ou des difficultés liées au déplacement de certains supports de test à l'intérieur du dispositif, ou bien des difficultés liées au vieillissement des supports de test (changement de couleur, etc.).

### Résumé de l'invention

La présente description propose donc un procédé automatique de contrôle de l'injection d'un liquide sur un support de test, à l'aide d'un injecteur et d'un capteur optique.

Selon un aspect, la description propose un procédé de contrôle d'injection d'un liquide sur un support test, le support de test comprenant un matériau absorbant apte à absorber le liquide et un réactif apte à réagir avec au moins un composant présent dans le liquide, le procédé étant mis en œuvre à l'aide d'un injecteur et d'un capteur optique, le procédé comprenant :
- l'injection du liquide sur le matériau absorbant du support de test par l'injecteur,
- le calcul d'une variation d'une valeur d'une propriété physique (la variation étant liée à l'injection de liquide sur le matériau absorbant) à l'aide du capteur optique d'une région de contrôle du support de test durant l'injection,
- en réponse à la détermination que la variation est inférieure à un seuil, fin de l'injection, et/ou
- en réponse à la détermination que la variation est supérieure à un seuil (le même seuil), continuation de l'injection de liquide sur le support de test.

Dans un mode de réalisation, le calcul de la variation est effectué à l'aide d'au moins deux valeurs de la propriété physiques prises espacées dans le temps avec de l'injection de liquide entre les deux obtentions. En particulier, le procédé peut comprendre une obtention d'une valeur relative à la propriété physique avant l'injection.

Dans un mode de réalisation, l'injection est effectuée par séquences d'injection intermittente et la variation de la valeur est calculée à partir d'au moins une valeur obtenue avant une séquence d'injection et au moins une valeur obtenue après la séquence d'injection.

La valeur obtenue avant une séquence d'injection peut être obtenue par une moyenne de plusieurs valeurs obtenues avant injection.

D'une façon générale, la moyenne de plusieurs valeur peut être une moyenne temporelle (sur plusieurs images successives) et/ou spatiales (sur toute la région de contrôle ou une portion de celle-ci).

Dans ce mode de réalisation, un nombre maximal de séquences d'injection peut être prédéfini.

Dans un exemple, au moins trois séquences d'injections ont lieu et au moins deux calculs de la variation ont lieu avant la fin de l'injection.

Dans un mode de réalisation, l'injection est continue et le calcul de la variation se fait en continu.

Dans un mode de réalisation du support de test, le matériau absorbant définit une voie d'acheminement du liquide (« *pathway* » en anglais) depuis une région d'injection, qui reçoit le liquide de l'injecteur jusqu'à la région de contrôle, dans lequel les réactifs se trouvent, le long de la voie d'acheminement, entre la région d'injection et la région de contrôle.

Dans un mode de réalisation, la région de contrôle est une région du matériau absorbant, par exemple une région extrémale située au-delà des réactifs le long de la voie d'acheminement du liquide, et la valeur est relative à la couleur de la région de contrôle. Une fois saturée en liquide, la couleur de la région de contrôle évolue faiblement.

Dans un mode de réalisation, la région de contrôle est un pad colorimétrique du support de test. La propriété physique peut être la couleur du pad colorimétrique. Alternativement ou complémentairement, la propriété physique est une dimension du pad colorimétrique. En effet, le pad, en s'imbibant, s'élargit.

Le procédé peut en outre comprendre, en réponse à la fin de l'injection, analyse du support de test à l'aide du capteur optique.

Selon un aspect, la description se rapporte aussi à une station d'analyse de support de test, comprenant un injecteur et un capteur optique, la station d'analyse étant configurée pour mettre en œuvre un procédé tel que décrit précédemment.

Dans un mode de réalisation, le capteur optique est configuré pour voir le support de test en entier, c'est-à-dire à tout le moins une face du support de test.

Selon un aspect, la description se rapporte aussi à un dispositif d'analyse d'urine comprenant une station telle que décrite précédemment, et un support de test, dans lequel le support de test comprend un matériau absorbant apte à absorber le liquide et un réactif apte à réagir avec au moins un composant présent dans le liquide.

Selon un aspect, la description se rapporte à un produit programme d'ordinateur comprenant des instructions aptes à mettre en œuvre un procédé tel que décrite précédemment lorsque les instructions sont exécutées par un processeur, notamment un processeur d'une station telle que décrite.

### Présentation des figures

D'autres caractéristiques, détails et avantages apparaîtront à la lecture de la description détaillée ci-après, et à l'analyse des dessins annexés, sur lesquels :
- [FIG. 1] : la figure 1 présente une représentation schématique et simplifiée d'un dispositif d'analyse d'urine installé dans une cuvette de toilettes,
- [FIG. 2] : la figure 2 présente une vue éclatée du dispositif d'analyse d'urine, dans laquelle la station et la cartouche sont visibles,
- [FIG. 3] : la figure 3 présente un représentation schématique d'un circuit fluidique d'une station de mesure,
- [FIG. 4] : la figure 4 présente une vue en coupe d'une cartouche et d'une station selon un mode de réalisation, à l'emplacement d'un analyseur optique de la station,
- [FIG. 5] : la figure 5 présente un mode de réalisation d'un support de test, avec l'extrémité injection illustré et le camp de vision d'un capteur optique qui sont représenté,
- [FIG. 6] : la figure 6 présente l'évolution temporelle d'un support de test schématiquement (A) et en réel (B),
- [FIG. 7] : la figure 7 présente un diagramme d'un procédé conforme à un mode de réalisation de l'invention, et
- [FIG. 8] : la figure 8 présente une architecture schématique d'une station de mesure et son écosystème.

### Description détaillée

La présente description présente un procédé de contrôle d'injection d'un liquide (par exemple de l'urine) sur des supports de test. Ce procédé peut être mis en œuvre à l'aide d'une station comprenant un injecteur et un capteur optique. La station reçoit typiquement le ou les supports de test à l'aide d'une cartouche qui loge une pluralité d'entre eux, pour former un dispositif de test. Dans un mode de réalisation, le dispositif est dimensionné pour être disposé sur une paroi d'une cuvette de toilette. Les documents suivants décrivent un exemple d'un tel dispositif d'analyse : WO2021175909 et WO2021175944, WO2023036805, WO2023036806, WO2023036808, WO2023036809. On parlera par la suite de documents WO pour les désigner d'une façon générale.

### Description de la station et du dispositif d'analyse

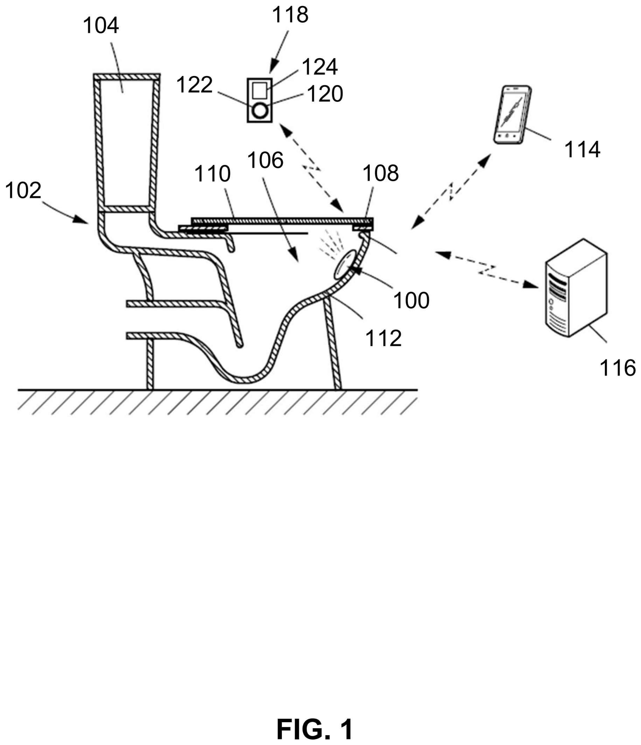

La figure 1 illustre schématiquement un dispositif d'analyse 100 (appelé aussi "dispositif 100" par la suite) pour l'analyse d'urine installé dans les toilettes 102. Les toilettes 102 comprennent généralement un réservoir d'eau 104, une cuvette 106, un siège 108 et un couvercle de siège 110. Le dispositif d'analyse 100 est notamment configuré pour être placé entièrement dans la cuvette des toilettes mais il peut toutefois être positionné ailleurs. Par "dans la cuvette", on entend "placé dans le volume intérieur défini par la cuvette". Le dispositif d'analyse 100 est disposé de manière amovible dans les toilettes 102. Par exemple, le dispositif d'analyse 100 peut être facilement retiré des toilettes pour remplacer une cartouche, puis replacé dans les toilettes 102. Le dispositif d'analyse 100 est placé sur une paroi 112 de la cuvette 106 des toilettes. Le dispositif d'analyse 100 est placé de telle sorte qu'il se trouve généralement sous le jet d'urine d'un utilisateur, de sorte que lorsqu'un utilisateur urine (généralement en position assise), l'urine entre en contact avec le dispositif d'analyse 100. Le dispositif d'analyse 100 peut communiquer à distance avec une entité distante, telle que le smartphone 114 ou un serveur 116.

Toutefois, en variante, la station peut être arrangée à l'extérieur des toilettes.

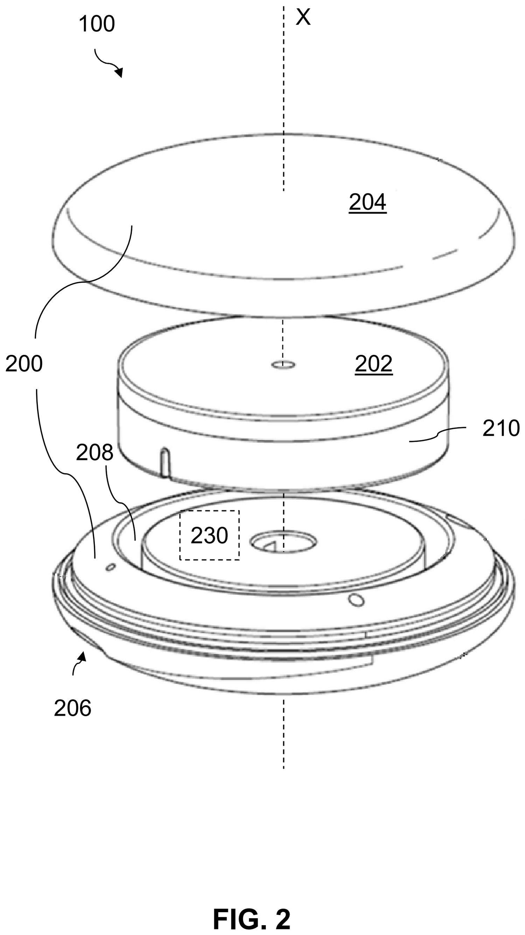

Comme l'illustre plus en détail la figure 2, le dispositif d'analyse 100 peut comprendre une station d'analyse d'urine 200 (appelée aussi « station 200 » par la suite) et une cartouche 202, montée de manière amovible sur la station 200. La cartouche 202 comprend du réactif apte à réagir avec de l'urine (appelé « réactif urinaire »). Dans un mode de réalisation sans cartouche, le dispositif d'analyse 100 et la station d'analyse 200 sont confondus.

Alternativement, la station 200 comprend directement du réactif urinaire sans partie amovible.

Plus de détail sur ce boitier sont données dans les documents WO.

La station 200 comprend un boitier 204 étanche, dont la fonction est aussi de récupérer l'urine pour l'acheminer jusqu'à un orifice de collecte 206, situé sur le boitier 204.

Le boitier 204 peut avoir un diamètre, mesuré dans la direction orthogonale à l'axe X, compris entre 50 mm et 150 mm. Le boitier 204 peut avoir une épaisseur, mesurée dans la direction de l'axe X, comprise entre 15 mm et 50 mm. Ainsi, le boitier 204 est suffisamment compact pour être entièrement logé dans la cuvette des toilettes. Le dispositif d'analyse d'urine 100 est discret. De plus, le boitier 204 est suffisamment grand pour entrer systématiquement en contact avec l'urine reçue dans la cuvette. L'utilisateur peut alors uriner dans les toilettes sans se soucier du dispositif d'analyse d'urine, ou alternativement viser sommairement.

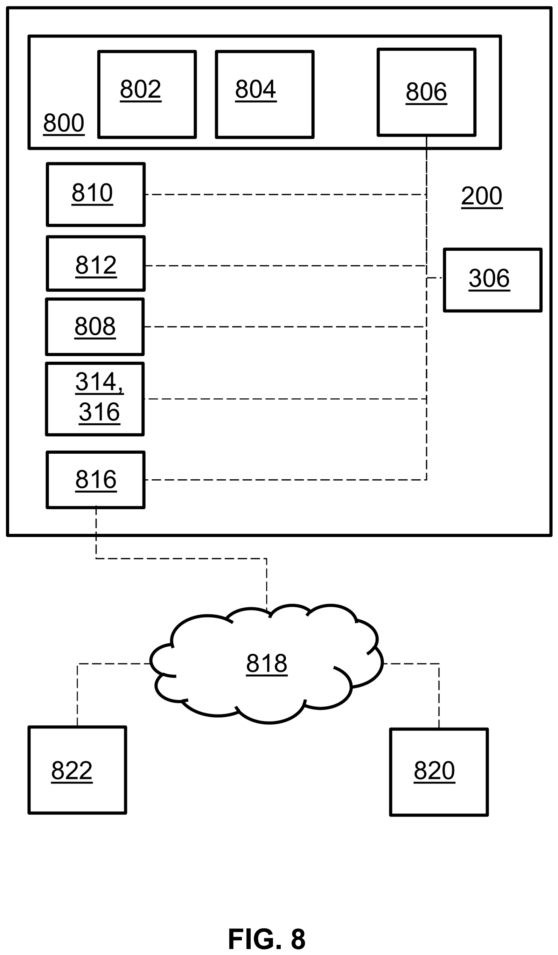

La station 200 comprend une circuiterie de contrôle 800, illustrée en figure 8, apte à contrôler les différents composants du dispositif 100, tels que la position d'une extrémité d'injection ou encore une activation d'une pompe ou encore, le cas échéant, d'une valve, comme cela sera décrit en détail par la suite.

### Logement pour cartouche

La station 200 comprend typiquement un compartiment annulaire 208, situé à l'intérieur du boitier 204, disposé autour d'un axe de rotation X et configuré pour recevoir une portion cylindrique 210 de la cartouche.

Dans le mode de réalisation des figures, la cartouche 202 comprend du réactif urinaire, notamment au moyen d'une pluralité de supports de test qui comprennent chacun au moins un réactif urinaire, par exemple un réactif sec. Dans l'exemple illustré, la pluralité de supports de test est disposée le long d'un cercle ou d'un arc de cercle autour de l'axe de rotation X et forment la pluralité de régions d'analyse. Dans un mode de réalisation, les supports de test sont des bandelettes de test. Les supports de test peuvent être enfermés, par exemple individuellement, dans une chambre étanche.

Le compartiment annulaire 208 s'étend typiquement sur 360° et forme une gorge configurée pour recevoir au moins partiellement la cartouche 202.

Le document EP4338839 décrit une méthode pour obtenir des chambres étanches dans une cartouche.

### Circuit fluidique

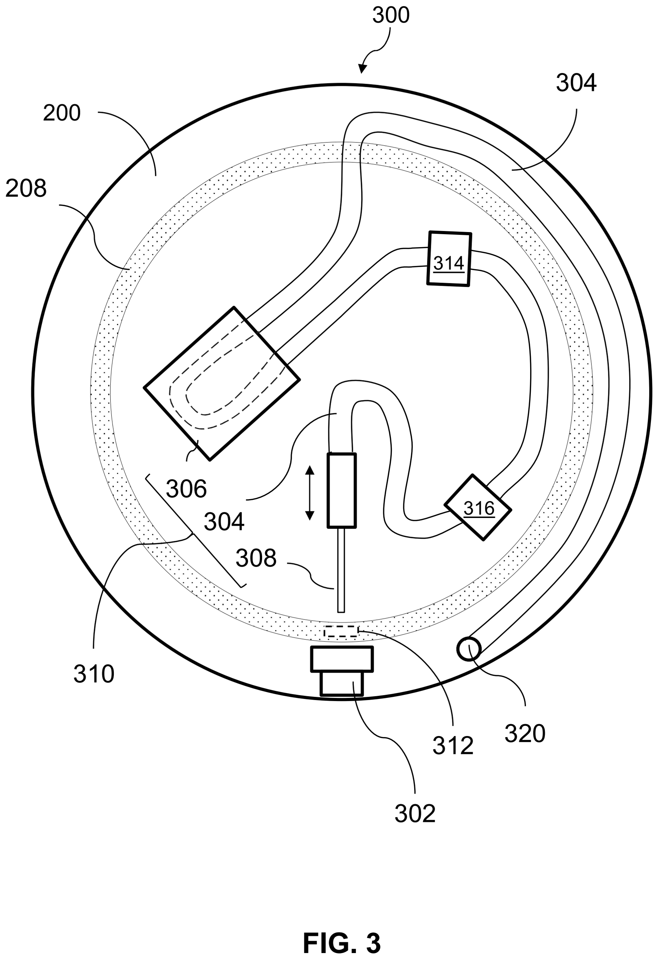

Pour acheminer l'urine qui ruisselle sur le boitier 204 jusqu'au support de test, la station d'analyse d'urine 200 comprend un circuit fluidique 300 illustré en figure 3.

Le circuit fluidique comprend un réservoir 302 (par exemple formé au niveau de l'orifice de collecte 206 pour récolter l'urine ruisselant sur le boitier 204), de la tuyauterie 304, une pompe 306, une extrémité d'injection 308. Le circuit fluidique peut comprendre également un orifice de purge 320 configuré pour drainer le liquide hors du dispositif 100.

La tuyauterie relie le réservoir 302, la pompe 306, l'extrémité d'injection 308 et éventuellement l'orifice de purge 320.

Dans le mode de réalisation illustré en figure 3, l'extrémité d'injection 308 se situe au niveau du réservoir 302 (logique LIFO ("last in - first out" en anglais, ou dernier entré - premier sorti en français). Dans une variante non illustrée, le réservoir 302 peut être séparé de l'extrémité d'injection 308 (logique FIFO ("first in - first out" en anglais, ou premier entré - premier sorti en français).

Sur la figure 3, un support de test 312 est représenté (en pointillé, car la figure 3 illustre une station sans la cartouche, et non à l'échelle) pour indiquer où ce dernier se trouve par rapport à la station. Le support de test 312 a été ici isolé des autres supports de test et de la cartouche.

Pour plus de détails, voir les documents WO2023036805, WO2023036806, WO2023036808, WO2023036809, ou encore les documents FR2410306, FR2410307 et FR2410308 (numéros de dépôt) déposés par Withings en 2024.

Dans un mode de réalisation, la station 200 comprend deux capteurs 314, 316 de présence de fluide espacés le long du circuit fluidique 300 et définissant ainsi une section de référence (dont le volume prédéterminé est connu par une circuiterie de contrôle 800). En mesurant le temps de déplacement du fluide entre les deux capteurs 314, 316 de présence de fluide, la circuiterie de contrôle 800 peut calculer le débit de la pompe 306.

Le ou les deux capteurs 314, 316 de présence de fluide peuvent comprendre des électrodes ou des sondes optiques. Le document WO2022184984 décrit en détail de tels capteurs (notamment le capteur optique).

L'extrémité d'injection 308, la pompe 306 et la tuyauterie 304 forment un injecteur 310. L'extrémité d'injection 308 peut comprendre une aiguille.

La pompe 306 peut avoir différents types de fonctionnement qui vont impacter le fonctionnement de l'injecteur 310. Par exemple, l'injecteur 310 peut fonctionner par séquence d'injection, c'est-à-dire que la pompe est activée par séquence, de sorte que le débit de liquide de l'injecteur 310 est intermittent (par exemple tous les microlitres, la pompe s'arrête). Alternativement, l'injecteur 310 peut fonctionner en continu, c'est-à-dire que la pompe est activée en continu, de sorte que le débit de liquide de l'injecteur 310 est ininterrompu.

Pour ce fonctionnement, la pompe 306 peut fonctionner par « coups de pompe », notamment lorsque la pompe 306 est péristaltique. Un coup de pompe est une brève activation contrôlée de la pompe 306. Par exemple des coups de pompes intermittent peuvent générer l'injection par séquence d'injection (avec notamment la correspondance entre un coup de pompe et une séquence d'injection) et des coups de pompes continus peuvent générer l'injection continue.

L'injecteur 310 est pilotée par une circuiterie de contrôle 800, présentée en fin de description.

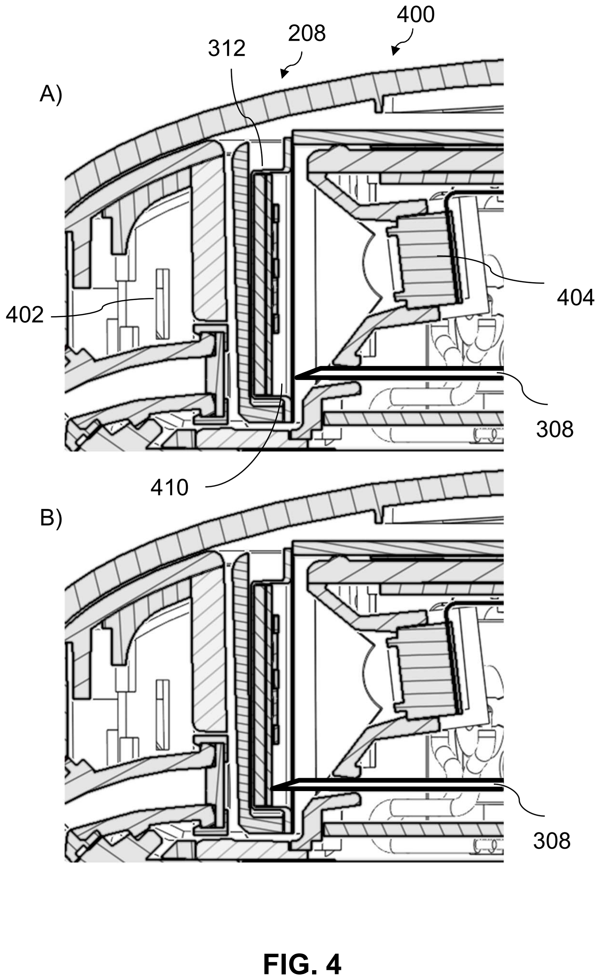

Comme illustré en figure 4, qui illustre une vue en coupe, l'extrémité d'injection 308 peut être déplacée entre plusieurs positions. On distingue notamment une position neutre (figure 4 A) durant laquelle l'extrémité d'injection 308 ne coopère pas le support de test 312 (donc notamment ne traverse pas la chambre étanche 410) et une position d'injection (figure 4 B) durant laquelle l'extrémité d'injection 308 pénètre dans la chambre étanche 410 et vient au contact, ou quasiment au contact du support de test 312, dans le but d'injecteur de l'urine sur le support de test.

En position neutre, l'extrémité d'injection 308 est, dans le mode de réalisation illustré sur les figures, située radialement à l'intérieur de la chambre étanche 410. Cela permet de maximiser le rayon du compartiment annulaire tout en minimisant la taille de la station 200.

Le dispositif 100 n'est toutefois qu'un exemple de réalisation concrète d'un dispositif d'analyse d'urine automatisé.

### L'analyseur

La station comprend en outre un analyseur 400, visible en figure 4.

L'analyseur 400 comprend une source lumineuse 402 (par exemple, une DEL) et au moins un capteur optique 404 sous la forme ici d'une caméra, pour détecter notamment un changement de couleur (en RVB par exemple) ou d'intensité. Sur l'exemple illustré, la lumière va de la source lumineuse 402 au capteur optique 404 en traversant la cartouche 202, le support de test 312 et donc notamment le réactif urinaire sur le support de test 312 (éclairage par transmission donc). Alternativement, la source lumineuse peut être du même côté que le capteur optique (éclairage par réflexion). Alternativement, le capteur optique 404 mesure à l'aide de la lumière ambiante, sans source lumineuse 402 dédiée.

Dans un mode de réalisation, le capteur optique 404 peut voir tout le support de test 312 (c'est-à-dire une face du support de test 312 tout entière). Dit autrement, le champ de vision du capteur optique 404 inclut tout le support de test 312 (au moins une face du support de test tout entière)

L'analyseur 400 est configuré pour obtenir une information relative à l'urine dans la région d'analyse, que ce soit une information directement obtenue à partir de l'urine ou une information indirectement obtenue à partir de l'urine (via le réactif).

### Les supports de tests

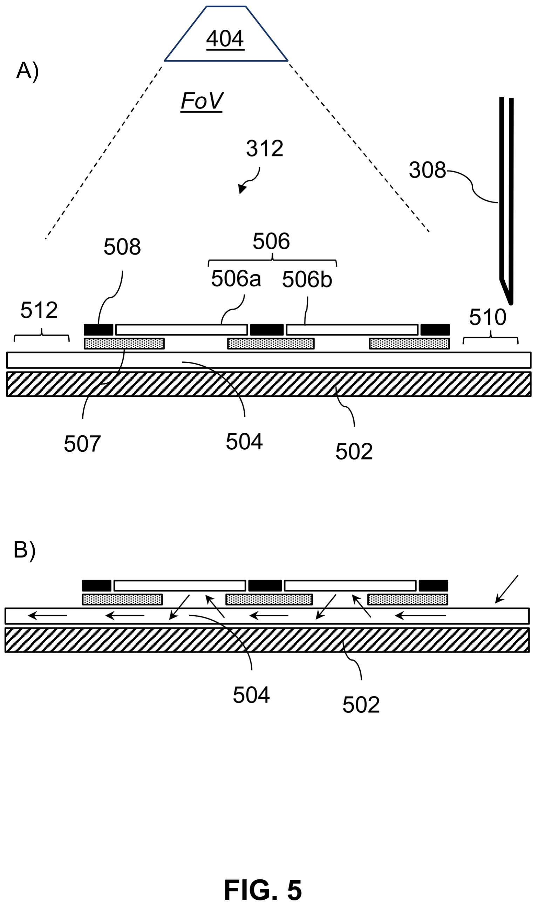

Le figure 5 illustre un mode de réalisation de support de test 312. Le support de test 312 comprend typiquement un châssis 502 (par exemple une bande en plastique pour rigidifier le support de test 312), sur lequel est montré un matériau absorbant 504 formant une voie de cheminement de liquide (appelée voie de cheminement, ou « *pathway* » en anglais). Le matériau absorbant peut comprendre de la cellulose.

Dans le mode de réalisation illustré sur la figure 5, le support de test 312 est de type colorimétrique, c'est-à-dire qu'il comprend en outre une région d'analyse 506, avec notamment un ou plusieurs pads colorimétriques 506a, 506b comprenant un réactif dont la couleur change lorsque le pad est mis en contact avec un composant du liquide (de sorte que le pad soit sensible au pH, à la gravité spécifique, aux cétones, à la vitamine C, etc.). La région d'analyse 506 peut être attachée au matériau absorbant 504 par au moins un adhésif 507. Un ou des masques 508 peuvent être prévus pour améliorer l'analyse optique. Ces masques et des variantes associées sont décrites dans le document EP4339598.

Sur la figure 5 A), le capteur optique 404, son champ de vision FoV, et l'extrémité d'injection 308 sont représentés pour favoriser la compréhension (les échelles ne sont pas respectées).

La région d'analyse 506 est en lien fluidique avec le matériau absorbant 504, de sorte que l'urine peut passer de l'un à l'autre, et réciproquement.

La figure 5 B) illustre le cheminement de l'urine sur le support de test 312. A cet égard, le support de test 312 comprend une région d'injection 510, configurée pour être positionnée en regard de l'extrémité d'injection 308 de l'injecteur 310. Le support de test 312 comprend en outre une région extrémale 512, située au-delà des régions d'analyse 506 le long de la voie d'acheminement.

Pour imbiber la région d'analyse 506, il faut injecter suffisamment d'urine dans la région d'injection 510. En effet, du fait de la configuration avec la voie de cheminement qui amène le liquide qui doit réagir sur la région d'analyse 506, il y a un risque que le liquide reste sur la voie de cheminement en privilégiant le passage dans le matériau absorbant 504. Dès lors, pour s'assurer que la région d'analyse 506 a bien été imbibée, une option est de saturer la voie de cheminement, c'est-à-dire le matériau absorbant 504, en liquide. Une des difficultés est de savoir comment déterminer cette saturation dans le cas d'une injection de liquide automatisée, c'est-à-dire sans le contrôle d'un opérateur.

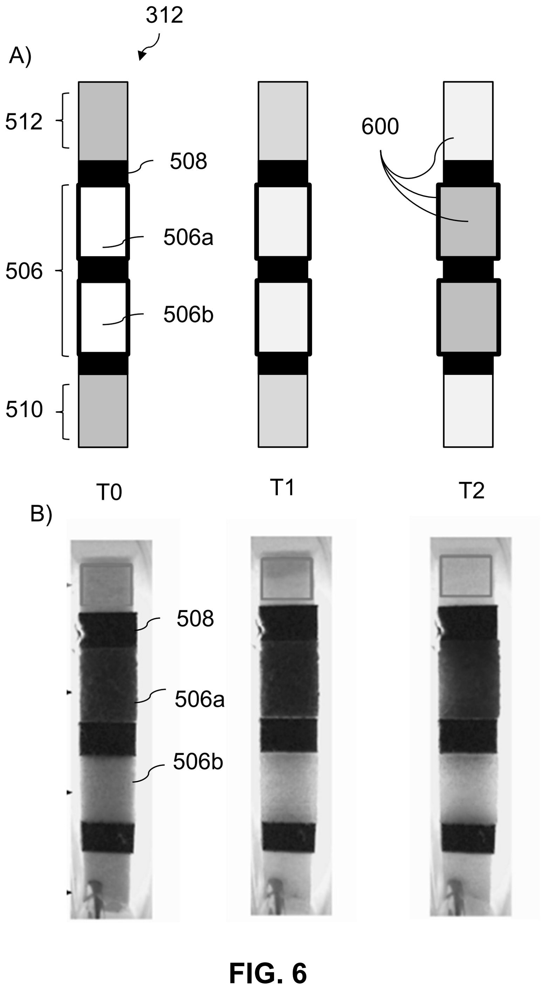

Afin de contrôler l'injection du liquide sur le support de test 312 par l'injecteur 310, le capteur optique 404 analyse une région du support de test 312, dite région de contrôle 600, dont plusieurs variantes sont illustrées sur la figure 6 et seront expliquées en détail.

### Le procédé de contrôle d'injection

Un procédé de contrôle d'injection automatique va être décrit. Il s'agit plus précisément d'un procédé de contrôle d'injection automatique. Il vise notamment, dans le cadre décrit précédemment, à s'assurer que suffisamment de liquide a été injecté. Le principe de ce procédé de contrôle n'est pas de détecter, via le capteur optique 404, un changement de couleur d'une région de la région de contrôle suite à l'injection de liquide par l'injecteur 310, comme cela est le cas avec des bandelettes de type « *lateral flow* » (où une ligne de contrôle apparait pour indiquer que le liquide a bien parcouru toute la voie d'acheminement), mais de détecter, à l'aide du capteur optique 404, le moment où une propriété physique de la région de contrôle 600, qui normalement évolue grâce à l'injection de liquide, cesse d'évoluer, malgré l'injection de liquide. Cela traduit une saturation en liquide et donc assure que la région de contrôle 600 a été correctement imbibée.

La figure 6 A) illustre en partie haute l'évolution temporelle d'un support de test avant injection (T0) et au cours de l'injection (T1 et T2). On a donc dans l'ordre temporel T0, T1 puis T2. La figure 6 B) représente, à des fins purement informatives, des échantillons réels, qui ont été schématisés en partie haute.

La figure 6 illustre un support de test 312 à trois temps différents T0, T1, T2, avec l'injection qui a début entre T0 et T1. L'injecteur 310 injecte du liquide sur la région d'injection 510. Le liquide se propage comme expliqué en relation avec la figure 5 B). La région de contrôle 600 du support de test 312 peut se situer à différents endroits.

Dans un mode de réalisation, la région de contrôle 600 correspond à la région extrémale 512, ou à une portion de la région extrémale. La région de contrôle 600 peut être un rectangle. Cette région extrémale 512 est formée par le matériau absorbant 504. Il n'y a pas de réactif particulier dans la région de contrôle comme dans les régions d'analyse, de sorte que la région extrémale 512 ne comprend donc pas de réactif à un composant du liquide. En revanche, le matériau absorbant 504 possède des propriétés physiques qui peuvent évoluer avec l'imbibition du liquide, comme sa transparence, et donc, sa couleur. En s'imbibant, le matériau absorbant 504 devient plus transparent, laissant ainsi passer davantage de lumière et apparaissant comme plus clair du fait de la source lumineuse 402. Une fois saturé en liquide, la transparence du matériau absorbant n'évolue plus et donc sa couleur n'évolue plus. La propriété physique, dont une valeur est mesurée par le capteur optique, peut donc être une couleur (par exemple le canal rouge du capteur RGB). La figure 6 A) illustre une variation de la couleur de la zone extrémale 512 à T0, T1 et T2, qui va en s'éclaircissant. La valeur mesurée peut être obtenue à partir d'une moyenne spatiale de la région de contrôle 600 (ou une portion de celle-ci) sur une image, mais aussi à partir, en combinaison, d'une moyenne temporelle, sur plusieurs images (notamment la moyenne temporelle des moyennes spatiales).

Dans un autre mode de réalisation, la région de contrôle 600 correspond à la région d'analyse 506, qui est formée par au moins un pad colorimétrique 506a, 506b. Deux variantes de ce mode de réalisation vont être présentées.

Dans une variante de ce mode de réalisation, la région de contrôle 600 correspond à la région d'analyse 506 ou à une portion de la région d'analyse 506. La région de contrôle 600 peut être un rectangle. La propriété physique est la couleur de la région d'analyse 506. En s'imbibant, la région d'analyse 506 réagit avec le liquide et change de couleur. Une fois la région d'analyse 506 saturée en liquide, la couleur du pad colorimétrique n'évolue plus. La propriété physique, dont une valeur est mesurée par le capteur optique, peut donc être une couleur (par exemple le canal rouge du capteur RGB). La valeur mesurée peut être obtenue à partir d'une moyenne spatiale de la région de contrôle 600 (ou une portion de celle-ci) sur une image, mais aussi à partir, en combinaison, d'une moyenne temporelle, sur plusieurs images (notamment la moyenne temporelle des moyennes spatiales).

Dans une autre variante de ce mode de réalisation, la propriété physique est la superficie de la région d'analyse 506 et notamment du pad colorimétrique 506a, 506b. En s'imbibant, la région d'analyse 506 se gorge de liquide et grossit, ce qui se traduit par une expansion de la superficie dans un plan parallèle au support de test 312 et donc une augmentation de sa surface. La figure 6 A) illustre la variation de la superficie de la région d'analyse 506 à T0, T1 et T2, qui va en s'agrandissant. Le capteur optique 404 peut mesurer un nombre de pixels saturés (du fait de la source lumineuse 402) à proximité de la région d'analyse 506 car ce nombre de pixels saturés diminue avec l'agrandissement de la superficie la région d'analyse 506. Alternativement, le capteur optique 404 peut mesurer le nombre de pixels plus foncés qui forment la région d'analyse 506.

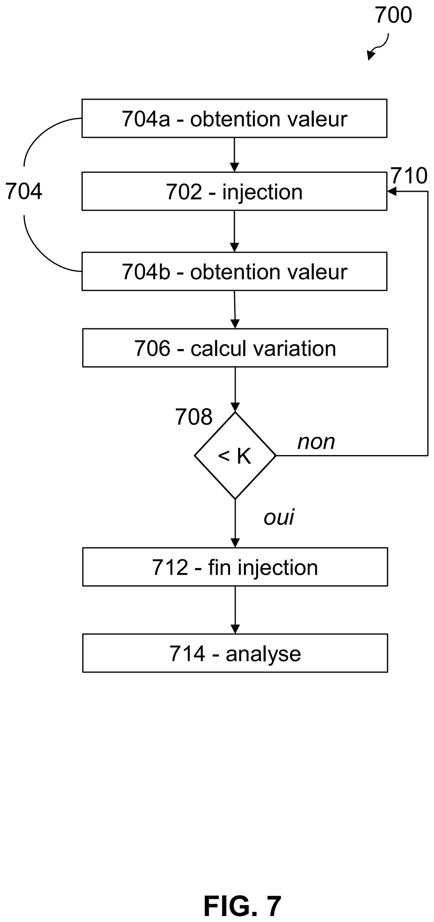

Un procédé 700 de contrôle d'injection automatique est représenté sur la figure 7. L'injecteur 310 est piloté par la circuiterie de contrôle 800 décrite en figure 8 par la suite.

Dans une étape 702, l'injecteur 310 injecte du liquide sur le matériau absorbant 504 du support de test 312. Typiquement, la circuiterie de contrôle 800 instruit l'injecteur 310 d'injecter. Dans une étape 706, la circuiterie de contrôle 800 calcule une variation d'une valeur physique relative à une propriété physique de la région de contrôle 600. La valeur physique est obtenue à l'aide du capteur optique 404, par exemple par traitement d'image (traitement de pixels). Pour calculer une variation de la valeur, il faut obtenir au moins deux valeurs de la propriété physique prises espacées dans le temps, avec de l'injection de liquide entre l'obtention des deux valeurs. Pour cela, le calcul de la variation 706 est précédé de l'obtention 704 de deux valeurs de la propriété physique espacées dans le temps (par exemple T0 et T1, ou T1 et T2, de la figure 6) avec de l'injection de liquide 702 entre les deux obtentions 704a, 704b. En particulier, le procédé 700 peut comprendre une étape d'obtention 704a d'une valeur de la propriété physique avant le début de l'injection 702 et donc avant l'imbibition du support de test (à sec donc). Une autre étape d'obtention 704b a lieu après que du liquide a été injecté. Alternativement, les étapes d'obtention d'une valeur 704 commencent après le début de l'injection 702.

Pour l'étape 706, la variation peut être une obtenue par un calcul de dérivée, de type dérivée discrète ou plus simplement un pourcentage de variation absolue.

Ensuite, dans une étape 708, la variation de la valeur calculée à l'étape 706 est comparée à un seuil K. Dans une étape 710, en réponse à la détermination que la variation est supérieure ou égale au seuil K, la circuiterie de contrôle 800 continue ou reprend l'injection de l'étape 702. Dans une étape 712, en réponse à la détermination que la variation (la variation de la valeur de la propriété physique donc) est inférieure au seuil K, la circuiterie de contrôle 800 arrête l'injection. Dans un mode de réalisation, le seuil K est inférieur à 5%.

Le procédé 700 de contrôle d'injection automatique comprend donc une boucle de contrôle qui fait continuer l'injection jusqu'à ce que les variations de la propriété physique de la région de contrôle 600 soient faibles, ce qui traduit une saturation de l'imbibition et signifie donc que l'injection peut être terminée.

En fonction du caractère continu ou intermittent de l'injection, l'obtention de valeur peut se faire quand il n'y a pas d'injection ou simultanément à une injection.

Dans le mode de réalisation par intermittence, l'injection est effectuée par séquences d'injection intermittente et une valeur du paramètre est obtenue avant une séquence d'injection et une valeur du paramètre est obtenue après la séquence d'injection. Le procédé comprend ainsi à la suite : obtention d'une valeur V1 du paramètre, injection #1 de liquide sur le support de test 312, obtention d'une valeur V2 du paramètre, calcul de la variation Δ(V1, V2), puis comparaison avec le seuil, et si Δ(V1, V2) > K, alors injection #2 de liquide sur le support de test 312, obtention d'une valeur V3 du paramètre, calcul de la variation Δ(V2, V3), puis comparaison avec le seuil, etc. En généralisant, le procédé comprend ainsi : obtention d'une valeur Vk du paramètre, injection #n, obtention d'une valeur Vk+1, calcul de la variation Δ(Vk, Vk+1), puis comparaison avec le seuil K, etc.

Par exemple, dans le mode de réalisation en continu, l'injection est effectuée en continu et le calcul de la variation se fait en continu, par exemple en utilisant des deux valeurs glissantes espacées d'un intervalle fixe dans le temps (par exemple quelques secondes).

Le nombre de fois que la circuiterie 800 effectue un calcul de la variation est au moins de deux, voire de cinq. Dit autrement, la boucle de contrôle est effectuée au moins deux, voire cinq fois. Dans le cas d'une injection continue, le calcul est effectué au moins pendant 5 secondes.

### Robustesse du procédé

La prise en compte de l'absence de (ou de la faible) variation de la valeur du paramètre physique permet d'obtenir un procédé applicable de façon générique, sans que le support de test ne prévoie des moyens exprès de contrôle de l'imbibition.

De plus, le procédé est robuste au vieillissement puisqu'elle permet de s'affranchir d'un critère absolu par rapport à la propriété physique (comme une couleur particulière ou autre). Dans le cas d'un dispositif d'analyse d'urine, les supports de test 312 peuvent être amenés à rester plusieurs mois dans un environnement humide et même s'ils arrangés dans une cavité étanche (voir les documents WO pour plus de détails), il se peut que les composants des supports de test 312 vieillissent. La comparaison se faisant entre deux images d'un même support de test, le procédé ne nécessite pas de calibration particulière ou complexe.

De la même façon, en fonction des types et des matériaux des supports de test, la couleur des supports de test 312 peut différer légèrement d'un support de test à l'autre, d'un batch à l'autre ou d'un fournisseur à l'autre. Le procédé est robuste à ce type de différence.

Le procédé est aussi robuste aux changements de débit de l'injecteur. A l'échelle de la station 200, il peut y avoir de grandes variabilités de débit, à cause de présence de bulles d'air dans le circuit fluidique 300 ou à cause du vieillissement de la pompe 306. Le procédé fonctionnant avec une boucle qui rétroagit, celle-ci est insensible à la perte de débit (voir plus de détails par la suite). Si le débit varie du simple au double en quelques jours, la durée de l'injection variera mais le résultat sera toujours similaire.

### Principe de sécurité

Dans un mode de réalisation, afin d'éviter de noyer la station, une mesure de sécurité est mise en place. Dans le cas d'un injecteur 310 fonctionnant par séquence d'injection, un nombre maximal de séquences d'injections est prédéfini, au-delà duquel l'injecteur 310 arrête l'injection. Par exemple, ce nombre peut être de trente coups de pompe. De la même façon, dans le cas d'une injection fonctionnant en continue, une durée maximale est prédéfinie, au-delà de laquelle l'injecteur 310 arrête l'injection, par exemple 10 secondes d'injection continue.

### Analyse du support de test

Après l'étape 712 (imbibition terminée), la circuiterie de contrôle 800 déclenche une analyse 714 du support de test 312, notamment de la région d'analyse 506 pour générer des données d'analyse à propos d'un paramètre du liquide injecté. Cette détermination se fait, dans un mode de réalisation, à l'aide du capteur optique 404. Le capteur optique sert donc à la fois pour l'analyse optique et pour le contrôle de l'injection. Le contrôle de l'injection s'effectue donc sans moyens additionnels, c'est-à-dire sans composant particulier dédié au contrôle de l'injection. En utilisant un capteur 404 qui voit l'intégralité d'une face du support de test 312, le même capteur optique 404 peut être utilisé pour contrôler l'injection et analyser le support de test 312.

### Architecture et circuiterie de contrôle

La figure 8 illustre schématiquement une station 200 avec une circuiterie de contrôle 800. La circuiterie de contrôle 800 comprend un processeur 802, une mémoire 804 (RAM ou ROM, par exemple permanente) et une interface I/O (« *in*/*out* » pour « entrée/sortie ») 806 pour échanger des données.

La mémoire 804 peut stocker des programmes exécutables par le processeur 802. Ces programmes mettent alors en œuvre un procédé tel que décrit précédemment.

La station 200 comprend en outre une batterie 808 configurée pour alimenter en énergie les composants électriques ou électroniques de la station 200.

La circuiterie de contrôle 800 peut notamment commander la pompe 306 pour piloter l'injection, ainsi qu'un moteur 810 pour déplacer la cartouche 202 dans la station 200, un moteur 812 pour déplacer l'extrémité d'injection 308 pour la mise en position d'injection (voir documents WO). La circuiterie de contrôle 800 peut notamment échanger des informations avec le ou les capteurs 314, 316 de présence de fluide.

La station 200 peut en outre comprendre un module de communication sans-fil 816 (par exemple un module *BlueTooth*, *BlueTooth Low Emission* et/ou un module Wi-Fi), relié à la circuiterie de contrôle 800. Le module 816 permet d'échanger des informations (émission et réception), via un réseau de communication 818 avec un terminal mobile 820 (par exemple un « *smartphone* ») et/ou un serveur distant 822. Le réseau de communication 818 peut être sans-fil et/ou filaire et/ou un mélange des deux. Les données d'analyse peuvent ainsi être envoyées au serveur 822 et ensuite transmise au terminal mobile 820 (ou bien communiquées directement au terminal mobile 820 qui les transmet ensuite au serveur). Inversement, la station 200 peut recevoir des mises à jour depuis le serveur distant 822 ou le terminal mobile 820, comme notamment un programme d'ordinateur tel que décrit précédemment.

### Variantes

La présente description a présenté un injecteur 310 avec une extrémité d'injection 308 et une pompe 306. Toutefois, l'injecteur peut prendre d'autres formes adaptées au dispositif dans lequel il est utilisé. L'injecteur, d'une façon générale, signifie un système permettant d'apporter un liquide de façon contrôlée.

## Revendications

1. Procédé de contrôle d'injection d'un liquide sur un support test (312), le support de test comprenant un matériau absorbant (504) apte à absorber le liquide et un réactif apte à réagir avec au moins un composant présent dans le liquide, le procédé étant mis en œuvre à l'aide d'un injecteur (310) et d'un capteur optique (404), le procédé comprenant :
- l'injection (702) du liquide sur le matériau absorbant (504) du support de test par l'injecteur (310),
- le calcul (706) d'une variation d'une valeur d'une propriété physique à l'aide du capteur optique (404) d'une région de contrôle (600) du support de test (312) durant l'injection,
- en réponse à la détermination (708) que la variation est inférieure à un seuil (K), fin de l'injection (712).

2. Procédé selon la revendication 1, dans lequel, en réponse à la détermination (708) que la variation est supérieure au seuil (K), continuation de l'injection (702) de liquide sur le support de test (312).

3. Procédé selon la revendication 1 ou 2, dans lequel :
- l'injection est effectuée par séquences d'injection intermittente, et
- la variation de la valeur est calculée (706) à partir d'au moins une valeur obtenue avant une séquence d'injection (704a) et au moins une valeur obtenue après la séquence d'injection (704b).

4. Procédé selon la revendication 3, dans lequel au moins trois séquences d'injections (702) ont lieu et au moins deux calculs de la variation (706) ont lieu avant la fin de l'injection (712).

5. Procédé selon l'une quelconque des revendications 1 à 2, dans lequel l'injection est continue et le calcul de la variation se fait en continu.

6. Procédé selon l'une quelconque des revendications 1 à 5, dans lequel matériau absorbant (504) définit une voie d'acheminement du liquide depuis une région d'injection (510), qui reçoit le liquide de l'injecteur (310) jusqu'à la région de contrôle (600), dans lequel les réactifs se trouvent, le long de la voie d'acheminement, entre la région d'injection (510) et la région de contrôle (600).

7. Procédé selon l'une quelconque des revendications 1 à 6, dans lequel la région de contrôle (600) est une région du matériau absorbant (504) et la valeur est la couleur de la région de contrôle (600).

8. Procédé selon l'une quelconque des revendications 1 à 7, dans lequel la région de contrôle (600) est un pad colorimétrique (506a, 506b) du support de test.

9. Procédé selon la revendication 8, dans lequel la propriété physique est la couleur du pad colorimétrique (506a, 506b).

10. Procédé selon la revendication 8, dans lequel la propriété physique est une dimension du pad colorimétrique (506a, 506b).

11. Procédé selon l'une quelconque des revendications 1 à 10, comprenant en outre :
- en réponse à la fin de l'injection (712), analyse (714) du support de test (312) à l'aide du capteur optique (404).

12. Station d'analyse (200) de support de test, comprenant un injecteur (310) et un capteur optique (404), la station d'analyse (200) étant configurée pour mettre en œuvre un procédé selon l'une quelconque des revendications 1 à 11.

13. Station selon la revendication 12, dans laquelle le capteur optique (404) est configuré pour voir le support de test (312) en entier.

14. Dispositif d'analyse d'urine comprenant une station selon la revendication 12 ou 13, et un support de test, dans lequel le support de test comprend un matériau absorbant (504) apte à absorber le liquide et un réactif apte à réagir avec au moins un composant présent dans le liquide.

15. Produit programme d'ordinateur comprenant des instructions aptes à mettre en œuvre un procédé selon l'une quelconque des revendications 1 à 11 lorsque les instructions sont exécutées par un processeur.
